# EUROPEAN PATENT APPLICATION

(11) **EP 1 671 638 A1**
(43) Date of publication of application: **21.06.2006**
(21) Application number: 06007027.3
(22) Date of filing: 18.09.2001
(51) Int. Cl.: A61K 31/663, A61P 19/10

(54) **Disphosphonate solutions**

(30) Priority: 18.09.2000 AU PR018900; 10.05.2001 AU PR485501
(62) Divisional of application: 01973807.9
(71) Applicant: F H Faulding & Co. Limited, Underdale, SA 5032 (AU)
(72) Inventor: Handreck, Gregory Paul, Ashwood Victoria 3147 (AU); Zhou, Wei, Ormond Victoria 3204 (AU); Tait, Russell John, Deepdene Victoria 3103 (AU)
(74) Representative: Baldock, Sharon Claire

(57) **Abstract**

A pharmaceutical product containing pamidronate and other diphosphonate solutions in an appropriate container, a pH of between 5 and 8 and without organic acid buffer or polyethylene glycol. The container may be treated glass or made of other appropriate material. Coated elastomeric stoppers are also included. A method of producing a pharmaceutical product comprising steps of making a suspension of pamadronic acid, adding sodium hydroxide, to form a solution adjusting the pH to between 5 and 8 and transferring the solution to a container.

## Description

### Field of the Invention

This invention relates to stable injectable solutions containing diphosphonates.

### Background

Various diphosphonic acids can be used as therapeutic active agents for the treatment of hypercalcaemia and in medication for the treatment of diseases such as osteoporosis and tumor osteolysis. ln a prepared solution, the active agent will be present as anions and is generally called diphosphonate, bisphosphonate or biphosphonate. An injection solution of diphosphonate can be prepared from the diphosphonic acid or one of its salts. A convenient method for administering these active agents is by intravenous infusion of prepared solutions into the bloodstream of a patient to be treated.

One diphosphonic acid, pamidronic acid is currently available in the form of a lyophilised product to be reconstituted prior to use. This reconstitution step involves not only both time and effort, but also introduces the possibility of adverse consequences through, for example improper reconstitution and mixing of the powder and contamination prior to administration. Previous efforts to formulate pamidronate solutions have suffered from stability problems with the solutions showing turbidity and loss of active product over time.

Glass has long been the material of choice for containers for pharmaceutical products. However, it has been found that diphosphonate solutions left in glass for extended periods display unacceptable levels of turbidity despite the good solubility and chemical stability of diphosphonates generally.

It is known that the level of turbidity of diphosphonate solutions in glass is affected by the pH of the solution, and that the level of turbidity decreases with increased acidity.

An approach to minimise the problem of reaction between the active substances and glass leachates is the use of excipients such as polyethylene glycols or acid buffers such as organic acids. Whilst the use of such excipients may assist, it is generally preferable to minimise the number of additional constituents of any injectable product solution.

### Summary of the Invention

Surprisingly, the inventors have found that it is possible to formulate stable diphosphonate solutions such as, in particular, pamidronate, which are neither highly acidic nor which involve the use of buffer systems. The inventors have found that solutions of diphosphonates of relatively neutral pH values do exhibit satisfactory stability provided appropriate containers are used.

This invention provides a stable and preprepared injectable solution of diphosphonate ready to be diluted by a practitioner administering the product to the patient. This enables the product to be provided in a consistent quality and avoids the need for the practitioner to reconstitute the active agent at the time of administration.

According to one aspect the present invention provides a pharmaceutical product comprising a container containing a diphosphonate in solution, wherein the solution:
(a) has a pH of between 5 and 8;
(b) is free of organic acid buffer and polyethylene glycol;
and wherein the container consists of at least one component manufactured from glass having at least a surface in contact with the solution, at least one said surface having been pre-treated to protect against the leaching of impurities from the glass by the solution.

According to a further aspect, the present invention provides a pharmaceutical product comprising a container containing a diphosphonate in solution, wherein the solution:
(a) has a pH of approximately 6.5; and
(b) is free of organic acid buffer and polyethylene glycol
and wherein the container consists of at least one component manufactured from glass having at least a surface in contact with the solution, at least one said surface having been pre-treated so as to protect against the leaching of impurities from the glass by the solution.

According to a further aspect, the present invention provides a pharmaceutical product comprising a container containing a diphosphonate in solution, wherein the solution:
(a) has a pH of between 5 and 8;
(b) is free of organic acid buffer and polyethylene glycol; and
wherein the container consists of at least one component manufactured from a non-glass material.

According to a further aspect the present invention provides a method of preparing a pharmaceutical product, said method comprising the steps of:
(a) preparing a suspension of pamodronic acid in water;
(b) adding sodium hydroxide solution to the suspension to obtain a second solution;
(c) adjusting the pH of the second solution to between 5 and 8; and
(d) transferring the second solution to a container.

### Description

In order to obtain adequate long-term stability, appropriate containers must be used for the solution of diphosphonate. Appropriate containers for this product include ampoules, vials, bottles, ready to use syringes and Shell Glass Vials.

It is believed that the principal cause of turbidity where glass containers have been used in the past is the leaching out from the glass of aluminium and/or other cations such as magnesium or calcium, depending upon the glass composition.

Where glass containers are used it is necessary to pre-treat the contact surface of the glass with an appropriate method to protect against the leaching of impurities from the glass by the solution. Preferably all potential contact surfaces will be appropriately treated. ln this way, the extent to which impurities leach from the glass over time is reduced. A preferred method of pre-treatment is a siliconization process using a one percent silicone solution to wash the vials, followed by double draining and heating at 310°C for thirty minutes. Vials pretreated in this manner are available from the French vial manufacturer Saint-Gobain Desjonqueres (SGD).

Other vial pretreatment techniques include the use of a high purity SiO₂ barrier formed on the inside vial surface by a plasma-deposition process. The process involves microwave energy being applied to a silicon containing precursor in the presence of oxygen. A plasma forms and a SiO₂ layer is formed on the glass surface from the gas phase. Vials pretreated in this manner are available from Schott.

In addition to treating the surface of the glass, it is also recommended to use containers which are made from glass having a low aluminium content. Glass typically used for pharmaceutical vials has in the order of 5 percent aluminium oxide. In order to reduce the problem of aluminium ion leaching, glass with lower aluminium content is recommended.

Where the solution is stored in a stoppered vial, the stopper provides a potential source of contamination. Typical elastomeric stoppers are potentially a source of metal ions eg calcium, zinc and magnesium ions which can react with the diphosphonate to form insoluble matter. In order to reduce the possibility of contamination, stoppers with low levels of these ions and other potential contaminants are to be used, preferably coated to form an inert barrier. An example of an appropriate stopper is the Daikyo D777-1 stopper. Daikyo D777-3 stoppers may also be used. Preferably the stopper has a low calcium, magnesium and ash content and is at least coated on the contact surface (being the surface of the stopper which when placed in a vial is exposed to the contents of the vial) with a fluorinated resin such as tetrafluoroethylene polymer, trifluorochloroethylene polymer, tetrafluoroethylene-hexafluoropropylene copolymer, fluorovinylidene polymer, vinylidene fluoride polymer, vinyl fluoride polymer, tetrafluoroethylene-ethylene copolymer, ethylene-tetrafluoroethylene copolymer, or perfluoroalkoxy polymer. It is more preferred that the stopper is coated with a fluorinated resin selected from a group consisting of tetrafluoroethylene polymer, trifluorochloroethylene polymer, tetrafluoroethylene-hexafluoropropylene copolymer, vinylidene fluoride polymer, vinyl fluoride polymer, and tetrafluoroethylene-ethylene copolymer. For example, the stopper can be a FluroTec® stopper manufactured by Daikyo and distributed by West Pharmaceuticals Services.

Containers, such as vials, may be constructed from any suitable other materials in addition to glass, such as polyethylene, polypropylene and polymethylpentene. For example, the vial could be constructed from Crystal Zenith® resin as manufactured by West Pharmaceuticals Services.

This invention is generally applicable to all diphosphonates. Specifically, this includes solutions of pamidronate, zolindronate, chlodronate, etidronate, alendronate and tiludronate. These can be prepared from their respective diphosphonic acid form or from a therapeutically acceptable salt form. The acids of the above diphosphonates are:- pamidronic acid [(3-amino-1-hydroxypropylidene) diphosphonic acid], zoledronic acid [(1)-hydroxy-2-(1H-imidazol-1-yl)ethylidene) diphosphonic acid]; chlodronic acid [dichloromethylene disphosphonic acid]; etidronic acid [(1-hydroxyethylidene) diphosphonic acid]; alendronic acid [(4-amino-1-hydroxy-butylidene diphosphonic acid]; and tiludronic acid [[((p-chloro-phenyl)thio)methylene] diphosphonic acid] respectively. This invention is particularly applicable to pamidronate and zolendronate.

It is preferred to have a product within the biological pH range i.e. of between about 5 and 8, to reduce the incidence of potential adverse reactions relating to acidic or alkaline solutions. Surprisingly it has been found that a stable solution can be produced having a pH of 5 - 8. A pH level of approximately 6.5 is preferred. At pH levels below about 5 there is a risk of producing venous type irritations and other unwanted side effects. pH levels above about 8 give rise to generally unacceptable levels of turbidity.

Solutions of diphosphonates will generally have a pH above that desired. For example, a solution of one percent pamidronate disodium salt in distilled water has a pH of approximately 8.3. The pH is adjusted with a suitable acid or alkali. Suitable acids include any acid such as hydrochloric or phosphoric acid. Phosphoric acid is preferred. Suitable alkalis include sodium hydroxide.

As the person skilled in the art will appreciate, other standard components, such as sugar alcohols and sodium chloride and water may be included in the solution, as required. Mannitol is the preferred sugar alcohol.

Pamidronate solutions are preferably prepared by slowly adding sodium hydroxide solution to a suspension of pamidronic acid in water in a 2:1 molar ratio of sodium hydroxide to pamidronic acid, adding mannitol if desired, mixing by stirring until both pamidronic acid and mannitol (if appropriate) are completely dissolved and adjusting the pH with phosphoric acid and if necessary sodium hydroxide solution. Preferably the preparation of the solutions is carried out under nitrogen. Other diphosphonate solutions can be prepared in analogous fashion.

### Example 1:

Preparation of pamidronate solution.

To a mixing vessel approximately 10% of the required amount of Water for Injection is added and then bubbled with nitrogen gas for at least 15 minutes. The sodium hydroxide, in an amount to give a 2:1 molar ratio to pamidronic acid is then added with stirring to dissolve and the solution cooled to less than 30°C.

A different closed mixing vessel is flushed with nitrogen gas for at least 15 minutes. Approximately 70% of the Water for Injection is added to the closed mixing vessel through a port and the mixing bubbled with nitrogen gas for at least 15 minutes. Pamidronic acid is then added to the mixing vessel with stirring and mixed for 5 minutes giving a suspension. The sodium hydroxide solution is then added over a 5 minute period with stirring to give a clear solution. Mannitol is then added to the solution with stirring for at least 5 minutes until dissolved. The pH is then checked and adjusted to a range of between 5 and 8 preferably, between 6.3 and 6.7 by addition of 1.0N phosphoric acid at the rate of approximately 12.1 g/L (calculated on total batch size) and if necessary 1.0N sodium hydroxide, whilst keeping the temperature between 35°C and 45°C. The volume is adjusted to the required level with Water for Injection and the solution cooled to below 30°C. The pH is then rechecked and adjusted if necessary to between 6.3 and 6.7, with 1.0N phosphoric acid or 1.0N sodium hydroxide if and as necessary.

### Example 2:

In this example the product solution was composed of the following:

| | |
|---|---|
| pamidronic acid | 2.53 mg |
| mannitol | 47.0 mg |
| sodium hydroxide | 0.43 mg |
| pH | qs to 6.3-6.7 using 1.0N sodium hydroxide or 1.0N phosphoric acid |
| Water for Injection | qs to 1.0 mL |

The formulated solution was filled into 10 mL siliconised, low aluminium, Type I glass vials, supplied by SGD. Each vial was enclosed by a 20 mm, S10-F451, D777-1, B2-40, FluroTec® stopper supplied by West Pharmaceuticals Services.

Table 1 .shows the test results measured over a 24 month period while being stored inverted at 25°C, relative humidity (RH) 60%.

**Table 1:**

| | Initial (0 months) | 6 months | 12 months | 18 months | 24months |
|---|---|---|---|---|---|
| Appearance | N | N | N | N | N |
| Potency | 98.7% | 99.9% | 99.8% | 100.1% | 99.1 |
| pH | 6.4 | 6.2 | 6.3 | 6.4 | 6.5 |
| Metal ions | | | | | |
| -silicon ppm | 0.6 | | 2.9 | | 2.9 |
| -calcium ppm | 0.09 | | 0.05 | | 0.07 |
| -aluminium ppm | 0.05 | | 0.12 | | 0.11 |

| | | | | | |
|---|---|---|---|---|---|
| N - Clear colourless solution, free from visible particles. | | | | | |

### Example 3:

In this example the product solution was composed of the following:

| | |
|---|---|
| pamidronic acid | 7.58 mg |
| mannitol | 37.5 mg |
| sodium hydroxide | 1.29 mg |
| pH | qs to 6.3-6.7 using 1.0N sodium hydroxide or 1.0N phosphoric acid |
| Water For Injection | qs to 1.0 mL |

The formulated solution was filled into 10 mL siliconised, low aluminium, Type I glass vials, supplied by SGD. Each vial was enclosed by a 20 mm, S10-F451, D777-1, B2-40, Fluro Tec® stopper supplied by West Pharmaceuticals Services.

Table 2 shows the test results measured over a 24 month period while being stored inverted at 25°C, relative humidity (RH) 60%.

**Table 2:**

| | Initial (0 months) | 6 months | 12 months | 18 months | 24 months |
|---|---|---|---|---|---|
| Appearance | N | N | N | N | N |
| Potency | 100.2% | 101.9% | 100.4% | 102.3% | 101.1 |
| pH | 6.4 | 6.3 | 6.4 | 6.3 | 6.5 |
| Metal ions | | | | | |
| -silicon ppm | 0.6 | | 6.2 | | 12.9 |
| -calcium ppm | <0.04 | | 0.1 | | 0.24 |
| -aluminium ppm | 0.06 | | 0.25 | | 0.56 |

| | | | | | |
|---|---|---|---|---|---|
| N - Clear colourless solution, free from visible particles. | | | | | |

### Example 4:

In this example the product solution was composed of the following:

| | |
|---|---|
| pamidronic acid | 2.53 mg |
| mannitol | 47.0 mg |
| sodium hydroxide | 0.86 mg |
| pH | qs to 6.3-6.7 using 1.0N sodium hydroxide or 1.0N phosphoric acid. |
| Water For Injection | qs to 1.0 mL |

The formulated solution was filled into 10 mL siliconised, low aluminium, Type I glass vials, supplied by SGD. Each vial was enclosed by a 20 mm, S10-F451, D777-1, B2-40, Fluro Tec® stopper supplied by West Pharmaceuticals Services.

Table 3 shows the test results measured over a 21 month period while being stored inverted at 25°C, relative humidity (RH) 60%.

**Table 3:**

| | Initial (0 months) | 6 months | 12 months | 18 months | 21 months |
|---|---|---|---|---|---|
| Appearance | N | N | N | N | N |
| Potency | 103.6% | 103.5% | 104.0% | 104.0 | 104.5 |
| pH | 6.5 | 6.4 | 6.5 | 6.6 | 6.5 |
| Metal ions | | | | | |
| -silicon ppm | 0.31 | 0.2 | 0.47 | - | - |
| -calcium ppm | 0.06 | <0.04 | <0.04 | - | - |
| -aluminium ppm | 0.17 | <0.04 | <0.04 | - | - |

| | | | | | |
|---|---|---|---|---|---|
| N - Clear colourless solution, free from visible particles. | | | | | |

### Example 5:

In this example the product solution was composed of the following:

| | |
|---|---|
| pamidronic acid | 7.58 mg |
| mannitol | 37.5 mg |
| sodium hydroxide | 2.58 mg |
| pH | qs to 6.3-6.7 using 1.0N sodium hydroxide or 1.0N phosphoric acid. |
| Water For Injection | qs to 1.0 mL |

The formulated solution was filled, into 10 mL siliconised, low aluminium, Type I glass-vials, supplied by SGD. Each vial was enclosed by a 20 mm, S10-F451, D777-1, B2-40, FluroTec® stopper supplied by West Pharmaceuticals Services.

Table 4 shows the test results measured over a 21 month period while being stored inverted at 25°C, relative humidity (RH) 60%.

**Table 4:**

| | Initial (0 months) | 6 months | 12 months | 18 months | 21 months |
|---|---|---|---|---|---|
| Appearance | N | N | N | N | N |
| Potency | 98.9% | 99.2% | 100.0% | 99.1 | 99.4 |
| pH | 6.5 | 6.4 | 6.5 | 6.6 | 6.5 |
| Metal ions | | | | | |
| -silicon ppm | 0.29 | 0.3 | 0.65 | - | - |
| -calcium ppm | 0.18 | 0.10 | 0.13 | - | - |
| -aluminium ppm | 0.12 | <0.04 | 0.07 | - | - |

| | | | | | |
|---|---|---|---|---|---|
| N - Clear colourless solution, free from visible particles. | | | | | |

In each of the examples 2 to 5 above, the solution was prepared by the process set out in Example 1.

It is understood that various modifications, alternatives and/or additions may be made to the product specifically described herein without departing from the spirit and ambit of the invention.

Throughout the description and claims of this specification the word "comprise" and variations of that word such as "comprises" and "comprising" are not intended to exclude other additives, components, integers or steps.

## Claims

1. A pharmaceutical product comprising a container containing a diphosphonate in solution said diphosphonate selected from the group consisting of pamidronate, zoledronate, chlodronate, etidronate, alendronate and tiludronate wherein the solution:
(a) has a pH of between 5 and 8; and
(b) is free of organic acid buffer and polyethylene glycol
and wherein the container consists of at least one component manufactured from glass having at least a surface in contact with the solution, at least one said surface having been pre-treated so as to protect against the leaching of impurities from the glass by the solution.

2. A pharmaceutical product according to any one of the preceding claims wherein the diphosphonate is pamidronate.

3. A pharmaceutical product according to any one of the preceding claims wherein the diphosphonate is zoledronate.

4. A pharmaceutical product according to any one of the preceding claims wherein the pH of the solution is approximately 6.5.

5. A pharmaceutical product comprising a container containing a diphosphonate in solution, wherein the solution:
(a) has a pH of approximately 6.5; and
(b) is free of organic acid buffer and polyethylene glycol
and wherein the container consists of at least one component manufactured from glass having at least a surface in contact with the solution, at least one said surface having been pre-treated so as to protect against the leaching of impurities from the glass by the solution.

6. A pharmaceutical product according to any one of claims 1 to 5 wherein the said surface has been pre-treated by a method which comprises:
(a) washing the said surface with a 1 % silicone solution;
(b) double draining the component; and
(c) heating the component at 310 degrees for 30 minutes.

7. A pharmaceutical product according to any one of claims 1 to 5 wherein the said surface has been pre-treated by application of a SiO₂ barrier by a plasma deposition process.

8. A pharmaceutical product according to any one of claims 1 to 7 wherein the glass component is manufactured from glass having an aluminium oxide content of less than 5%.

9. A pharmaceutical product comprising a container containing a diphosphonate in solution, wherein the solution:
(a) has a pH of between 5 and 8; and
(b) is free of organic acid buffer and polyethylene glycol
and wherein the container consists of at least one component manufactured from a non-glass material.

10. A pharmaceutical product according to claim 9 wherein the said component is manufactured from a material selected from the group consisting of polyethylene, polypropylene and polymethylpentene.

11. A pharmaceutical product according to either claim 9 or 10 wherein the pH is approximately 6.5.

12. A pharmaceutical product according to any one of the preceding claims wherein the container further comprises an elastomeric stopper.

13. A stable pharmaceutical product according to claim 12 wherein the stopper has a contact surface able to contact the solution within the container and wherein the contact surface of the elastomeric stopper is coated with a fluorinated resin.

14. A stable pharmaceutical product according to claim 13 wherein the fluorinated resin is selected from the group tetrafluoroethylene polymer, trifluorochloroethylene polymer, tetrafluoroethylene-hexafluoropropylene copolymer, fluorovinylidene polymer, vinylidene fluoride polymer, vinyl fluoride polymer, tetrafluoroethylene-ethylene copolymer, ethylene-tetrafluoroethylene copolymer, and perfluoroalkoxy polymer.

15. A stable pharmaceutical product according to claim 14 wherein the fluorinated resin is selected from the group tetrafluoroethylene polymer, trifluorochloroethylene polymer, tetrafluoroethylene-hexafluoropropylene copolymer, vinylidene fluoride polymer, vinyl fluoride polymer, and tetrafluoroethylene-ethylene copolymer.

16. A pharmaceutical product according to any one of the preceding claims wherein the solution further comprises mannitol.

17. A pharmaceutical product according to any one of the preceding claims wherein the solution further comprises sodium hydroxide.

18. A pharmaceutical product according to any one of the preceding claims wherein the solution further comprises phosphoric acid.

19. A method of preparing a pharmaceutical product comprising the steps of:
(a) preparing a suspension of pamodronic acid in water;
(b) adding sodium hydroxide solution to the suspension to obtain a second solution;
(c) adjusting the pH of the second solution to between 5 and 8; and
(d) transferring the second solution to a container.

20. A method of preparing a pharmaceutical product according to claim 19 wherein the amount of sodium hydroxide added to the slurry is in a 2:1 molar ratio to pamodronic acid.

21. A method of preparing a pharmaceutical product according to either claim 19 or 20, wherein the pH of the solution is adjusted to between 6.3 and 6.7.

22. A method of preparing a pharmaceutical product according to any one of claims 19 to 21, wherein mannitol is added to the solution.

23. A method of preparing a pharmaceutical product according to any one of claims 19 to 22, wherein the pH is adjusted using phosphoric acid.

24. A method of preparing a pharmaceutical product according to any one of claims 19 to 23, wherein the pH is adjusted using sodium hydroxide.

25. A pharmaceutical product according to any one of claims 1 to 18, prepared by a method according to any one of claims 19 to 24.

26. A pharmaceutical product substantially as described herein with reference to any one of the examples 2 to 5.

27. A method of preparing pharmaceutical product substantially as described herein with reference to any one of the examples.
